# EUROPEAN PATENT SPECIFICATION

(11) **EP 2 552 327 B1**
(45) Date of publication and mention of the grant of the patent: **09.08.2017**
(21) Application number: 11712138.4
(22) Date of filing: 25.03.2011
(51) Int. Cl.: A61B 17/22, A61B 17/30, A61B 17/29, A61B 17/00

(54) **DEVICE FOR POSITIONING AN IMPLANTED STRUCTURE TO FACILITATE REMOVAL**
VORRICHTUNG ZUR POSITIONIERUNG EINER IMPLANTIERTEN STRUKTUR ZUR LEICHTEREN ENTFERNUNG
DISPOSITIF DE POSITIONNEMENT DE STRUCTURE IMPLANTÉE POUR FACILITER SON ENLÈVEMENT

(30) Priority: 29.03.2010 US 318518 P
(43) Date of publication of application: 06.02.2013
(73) Proprietor: Cook Medical Technologies LLC, Bloomington, IN 47404 (US)
(72) Inventor: GOODE, Louis, B., Cranberry Township, PA 16066 (US); LUI, Chun, Kee, Apollo, PA 15613 (US); BOOKER, Robert, Vandergrift, PA 15690 (US)
(74) Representative: Ellis, Katherine Elizabeth Sleigh
(86) International application number: PCT/US2011/029915
(87) International publication number: WO 2011/123342

(56) References cited:
- US-A- 5 496 314
- US-A- 5 860 987
- US-A- 6 090 129
- US-A1- 2002 107 526
- US-A1- 2010 030 200

## Description

### BACKGROUND

1. Technical Field. This invention relates to a device for positioning an implanted elongated structure, such as an implanted electrical cardiac lead, to facilitate removal, or extraction, of the implanted elongated structure from the body of a patient.

2. Background Information. A variety of medical treatments and surgical methods entail implanting an elongated structure in the body of a human or veterinary patient. Examples of such elongated structures include catheters, sheaths, and cardiac electrical leads (such as pacemaker leads and defibrillator leads), as well as a variety of other devices. Over time, it can become necessary or desirable to remove the implanted elongated structure from the body of the patient. However, if the elongated structure has been implanted for an extended period of time, encapsulating biological tissue can grow around the elongated structure, making it difficult to remove the structure from the encapsulating tissue.

A heart pacemaker is typically implanted in a subcutaneous tissue pocket in the chest wall of a patient. A pacemaker lead extends from the pacemaker through a vein into a chamber of the patient's heart. The pacemaker lead commonly includes a conductor, such as an electrical wire coil, for conducting electrical signals (such as stimulating and/or sensing signals) between the pacemaker and the heart. Leads for defibrillators are generally similar to pacemaker leads, and are positioned about the heart. Defibrillator leads may be affixed either internally or externally of the heart.

While cardiac electrical leads typically have a useful life of many years, over time such leads may become encapsulated by fibrotic tissue against the heart itself or the wall of the vein, or against other surrounding tissue. Encapsulation is especially encountered in areas where the velocity of the flow of blood is low. The fibrotic tissue can be very tough, which makes it difficult to remove the lead from the area of the heart without causing trauma to the area. When small diameter veins through which a pacemaker lead passes become occluded with fibrotic tissue, separation of the lead from the vein can cause significant damage to the vein, including the possible dissection or perforation of the vein. In such cases, separation of the lead from the vein is usually not possible without restricting or constraining movement of the lead, *i.e.,* fixing the lead in position with respect to the patient, and in particular, with respect to the patient's vein.

To avoid this and other possible complications, some useless pacemaker or other leads are simply left in the patient when the pacemaker or defibrillator is removed or replaced. However, such a practice can incur the risk of an undetected lead thrombosis, which can result in stroke, heart attack, or pulmonary embolism. Such a practice can also impair heart function, as plural leads can restrict the heart valves through which they pass.

There are many other reasons why removal of a useless lead may be desirable. For example, if there are too many leads positioned in a vein, the vein can be obstructed to the extent that fluid flow through the vein is severely compromised. In addition, multiple leads can be incompatible with one another, thereby interfering with the pacing or defibrillating function. An inoperative lead can migrate during introduction of an adjacent second lead, and mechanically induce ventricular arrhythmia. Other potentially life-threatening complications can require the removal of the lead as well. For example, removal of an infected pacemaker lead may be desirable so as to avoid conditions such as septicemia or endocarditis.

Surgical removal of a heart lead in such circumstances may involve open heart surgery. However, open heart surgery is accompanied by significant risk and cost to the patient, as well as a potential for unintended complications. A variety of methods and apparatuses have been devised as alternatives to open heart surgery for heart lead removal. Several of these methods and apparatuses are described in related patents, such as U.S. Patent No. 5,697,936, titled "Device for Removing an Elongated Structure Implanted in Biological Tissue"; U.S. Patent No. 5,507,751, titled "Locally Flexible Dilator Sheath"; U.S. Patent No. 5,632,749, titled "Apparatus for Removing an Elongated Structure Implanted in Biological Tissue"; U.S. Patent No. 5,207,683, titled "Apparatus for Removing an Elongated Structure Implanted in Biological Tissue"; U.S. Patent No. 4,943,289, titled "Apparatus for Removing an Elongated Structure Implanted in Biological Tissue"; U.S. Patent No. 5,011,482, titled "Apparatus for Removing an Elongated Structure Implanted in Biological Tissue"; U.S. Patent No. 5,013,310, titled "Method and Apparatus for Removing an Implanted Pacemaker Lead"; U.S. Patent No. 4,988,347, titled "Method and Apparatus for Separating a Coiled Structure from Biological Tissue"; U.S. Patent No. 5,423,806, titled "Laser Extractor for an Implanted Object"; U.S. Patent No. 6,419,974, titled "Radio Frequency Dilator Sheath", and U.S. Patent Nos. 6,687,548 and 6,712,826, each titled "Apparatus for Removing an Elongated Structure Implanted in Biological Tissue", among others.

US 2002/0107526 discloses a medical grasping device for vascular use, having an outer sheath, an elongate control member extending within an outer sheath to a distal tip section, and a proximal control assembly including an actuation section joined to the elongate control member. Adjacent to the distal tip section is a grasping portion that is extendable from the outer sheath to create loops for grasping a target object for repositioning within the vascular system, or for removal from the patient, with loops being retractable into the outer sheath to hold the target object against the device during movement of the device. The elongate control member may be a cannula or tube having a lumen extending completely therethrough for placement over a guide wire already in the patient.

Most of the aforementioned patents describe manual, or mechanical, devices that are used for removing an implanted structure, such as a pacemaker lead. Others describe non-mechanical techniques, such as laser extraction and radio frequency extraction. The non-mechanical techniques have been effective in cases when the amount and/or placement of fibrous growth that surrounds the implanted lead renders manual extraction difficult or impossible. One example of an effective device that uses radio frequency extraction to enable the physician to cut away the heavy growth is the PERFECTA® electrosurgical dissection sheath, available from Cook Vascular Incorporated, of Leechburg, Pennsylvania. The PERFECTA® sheath utilizes an intermittent discrete RF dissecting arc between bipolar electrodes located at the sheath's distal end. This sheath enables the physician to separate, with directed precision, a transvenous lead from its fibrous binding attachments.

Although the prior art devices have been found to be reasonably effective in many situations, physicians continue to encounter particularly difficult situations in which existing extraction devices provide unsatisfactory or inconsistent results. Due to the multiplicity of factors that may contribute to the difficulty in extracting an implanted lead, a technique that may be effective in one instance, may not provide similarly successful results in another instance. For example, manual devices normally are provided with single or telescoping flexible sheaths. Such sheaths, generally formed from a polymer, are intended to have the flexibility to enable the sheath to traverse pathways in the vessel. However, such sheaths may lack sufficient strength to cut through particularly tough tissue growth and calcification around the implanted lead. They may also have difficulty traversing particularly tortuous pathways in the vessel. Laser and radio frequency employ energy to cut through fibrous growths. However, some growths may be too stubborn for even these energized sheaths. In addition, these sheaths may lack the flexibility to maneuver tortuous pathways.

In some instances, it is necessary to traverse a fairly tight curve in an artery or vein when tracking a lead intended for removal. One example is the curve in the subclavian vein. At times, removal along such a curve can be accomplished by maintaining good tension on the lead, e.g. with the help of a locking stylet well anchored to the distal end of the lead. This is intended to force the sheath to bend and thus negotiate the curve, without putting undue force upon the vein wall on the outside of the curve.

At other times, however, it can be difficult to negotiate the curved pathways with a lead removal device. In these and other instances, it would be desirable to have the ability to reposition the proximal end of the lead by directing the lead end away from the vessel having the tight curve, and into another vessel or pathway in a manner such that a less curved pathway is navigated when removing the lead. Thus, for example, when attempting to negotiate the curvature along the subclavian vein, it would be desirable to reposition the proximal end of the lead by redirecting it into the superior vena cava. In this case, a fairly straight pathway for lead removal may be achieved through the internal jugular vein.

### BRIEF SUMMARY

According to an aspect of the present invention, there is provided a device for adjusting a position of an elongated structure implanted in biological tissue to facilitate removal thereof as specified in claim 1.

A method is described for adjusting a position of an elongated structure implanted in biological tissue to facilitate removal thereof, wherein at least a portion of the elongated structure extends along a generally curved body pathway. A device comprising a sleeve member having a proximal end and a distal end, and a manipulator mechanism engaged with the sleeve member distal end and extendable in a distal direction therefrom is positioned for insertion along a substantially non-curved body pathway, wherein the manipulator mechanism is structured and arranged for capture of the elongated structure. A distal end of the device is inserted along the substantially non-curved body pathway, and the device is advanced along the substantially non-curved pathway until the device distal end approaches a length of the implanted elongated structure. The manipulator mechanism is maneuvered in a manner to capture the implanted elongated structure. The implanted elongated structure is locked onto the device, and the device having the elongated structure locked thereon is further advanced along the substantially non-curved pathway until the implanted elongated structure is at least substantially in the substantially non-curved pathway.

A device is described for adjusting a position of an implanted elongated structure implanted in biological tissue of a patient to facilitate removal thereof. An elongated shaft member has a proximal end and a distal end. A manipulator mechanism is engaged with the shaft member distal end and extendable in a distal direction therefrom. The manipulator mechanism is selectively maneuverable between a capture position wherein the implanted elongated structure is movably captured by the manipulator mechanism, and a locked position wherein movement of the implanted structure relative to the manipulator mechanism is at least substantially prevented. A control mechanism is engaged with the shaft member for controlling the maneuverability of the manipulator mechanism.

### BRIEF DESCRIPTION OF THE DRAWINGS

Fig. 1 is a perspective view of an embodiment of a device for use in adjusting the position of a portion of a cardiac lead to facilitate removal of the lead from the body of a patient;
Fig. 2 is a longitudinal sectional view of the device of Fig. 1;
Fig. 3 is a disassembled view of the device of Fig. 1, showing elements thereof prior to assembly;
Fig. 4 is a perspective view of the device of Fig. 1, wherein the handle has been removed to illustrate engagement of the plunger halves;
Fig. 5 is a side view of the interior surface of one of the handle halves of the device of Fig. 1;
Figs. 6-9 are side views illustrating various stages of operation of the device of Fig. 1;
Figs. 10-12 are enlarged views illustrating the positions of the manipulator arms 84, 86 in the positions depicted in Figs. 7-9, respectively, and showing a cardiac lead being captured and locked between the manipulator arms; and
Figs. 13-17 are schematic views illustrating use of the device of Fig. 1 for positioning a cardiac lead targeted for removal from the body of the patient.

### DETAILED DESCRIPTION

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

The present invention relates to a device for use in adjusting a position of an elongated structure that has previously been implanted in a patient, to facilitate removal of the elongated structure. In the following discussion, the terms "proximal" and "distal" will be used to describe the opposing axial ends of the device, as well as the axial ends of various component features of the device. The term "proximal" is used in its conventional sense to refer to the end of the device (or component thereof) that is closest to the operator during use of the device. The term "distal" is used in its conventional sense to refer to the end of the device (or component) that is at the greatest distance from the operator, or that is initially inserted into the patient.

The implanted structure targeted for positioning may comprise a cardiac lead. A cardiac lead, as the term is used herein, refers to a lead that is used in connection with a heart-related device. Non-limiting examples of cardiac leads that may be positioned by the inventive device include pacemaker leads, defibrillator leads, coronary sinus leads, and left ventricular pacing leads. When the device is used to position a cardiac pacemaker lead, the distal end of the cardiac lead will normally be located within the vascular system of the patient, and in particular, within a chamber of the patient's heart (such as in an atrium or ventricle of the heart). When the implanted elongated structure is a defibrillator lead, the distal end of the lead may be located either in or about the heart of the patient. The distal ends of other types of implanted elongated structures targeted for positioning may not necessarily be near the heart.

In addition to cardiac leads, the invention may also be used to position other devices or leads, such as neurological pacing and stimulation leads. A non-limiting list of still other structures that can be positioned by the inventive device includes implanted catheters, sheaths, cannulae and the like. For convenience, the following discussion will refer to the positioning of a cardiac lead, such as a pacemaker or a defibrillator lead, to facilitate removal of the lead from the body of a patient. However it should be understood that this is no way intended to be a limitation on the scope of the invention, and that the device may be suitable for positioning other implanted structures, such as the elongated structures referred to above.

When the inventive device is used for positioning a proximal end of a cardiac lead in order to facilitate eventual removal, or extraction, of the lead, those skilled in the art will appreciate that the proximal end of the lead should initially be severed from the control device, e.g., a pacemaker or a defibrillator, prior to any attempts to adjust the position of this severed lead end, for facilitating removal of the lead. Typically, the more distal portion of the lead will be implanted, and may be at least partially encapsulated by fibrotic tissue.

Generally speaking, the device for adjusting a position of an implanted elongated structure includes an elongated shaft mechanism, and a manipulator mechanism extendable from a distal end of the shaft mechanism. Preferably, the manipulator mechanism comprises a pair of controllable manipulator arms. A control mechanism enables the arms to be selectively manipulable between an "at rest", or free, position; a semi-locked, or "capture" position wherein the arms generally encircle or enclose the implanted structure but do not prevent movement of the arms or implanted structure relative to one another; and a locked position wherein the implanted structure is essentially locked into a fixed position relative to the arms, and further movement of the implanted structure relative to the arms is at least substantially prevented.

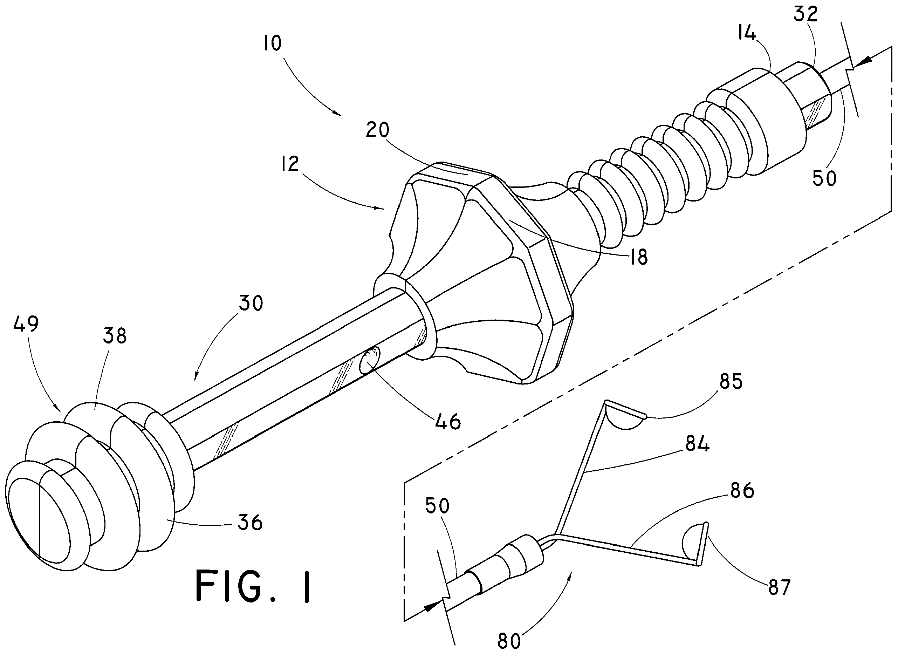

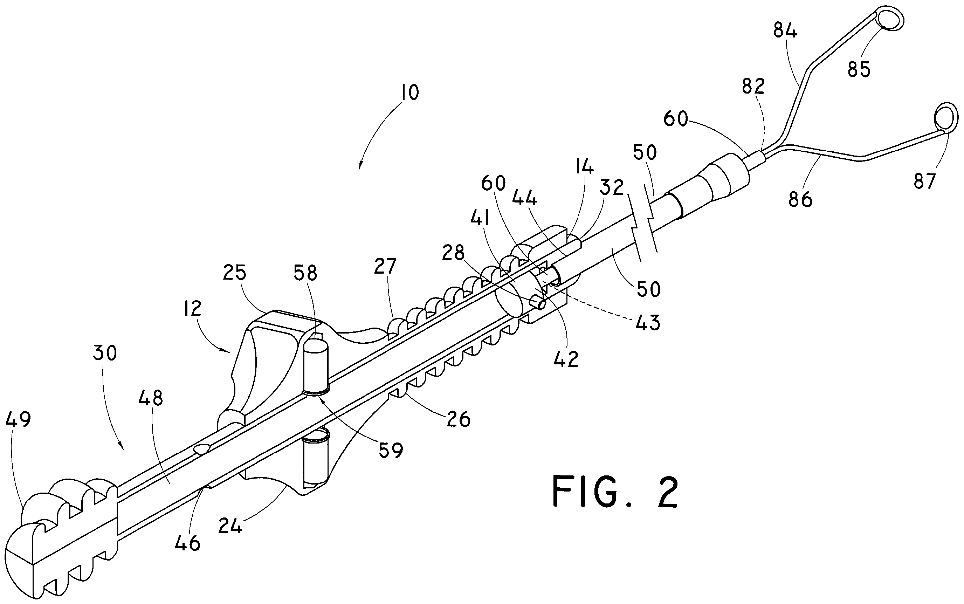

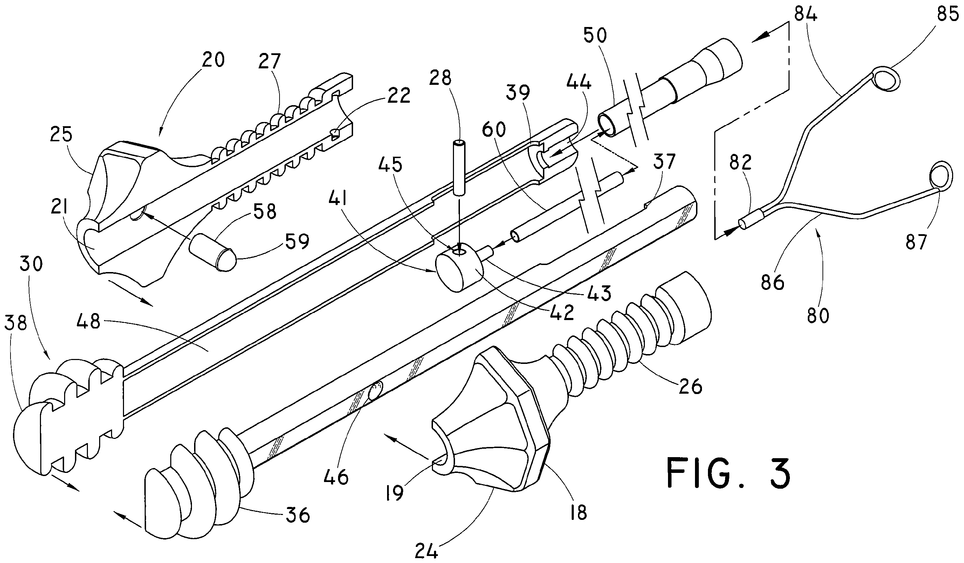

Fig. 1 depicts a perspective view of one embodiment of a positioning device 10 for use in adjusting a position of an implanted structure, such as a cardiac lead, to facilitate removal of the implanted structure from the body of a patient. Fig. 2 is a longitudinal sectional view of device 10, and Fig. 3 is a disassembled view of the device showing elements thereof prior to assembly.

In the embodiment shown in Fig. 1-3, positioning device 10 comprises a handle 12 and a plunger 30, wherein the plunger is movable relative to the handle. An outer sleeve 50 is engaged with distal end 32 of plunger 30, and is movable therewith. An inner sleeve 60, receivable within outer sleeve 50, is engaged with the distal end 14 of handle 12 and is movable therewith. A manipulator mechanism 80 for grasping and manipulating the cardiac lead extends from the distal end of inner sleeve 60.

In a preferred embodiment, manipulator mechanism 80 comprises a generally cylindrical base member 82 having manipulator arms 84, 86 extending in a distal direction therefrom. Preferably, manipulator arms 84, 86, comprise individual wire members that terminate in respective loops 85, 87, as shown. In one preferred embodiment, loops 85, 87 are filled with a material, such as solder. Filling the loops with a filler material as described provides increased visibility and tracking of the manipulator arms. In addition, the presence of a filler material ensures that the cardiac lead or other structure being positioned does not inadvertently become entangled or otherwise trapped within the loop. This structure also creates a less aggressive platform for the lead to be locked against, thus helping to prevent potential damage to a lead when in the locked position.

In the embodiment shown, handle 12 comprises first handle half 18 and second handle half 20. Plunger 30 comprises first plunger half 36 and second plunger half 38. Those skilled in the art will appreciate that handle 12 and plunger 30 need not necessarily be composed of first and second halves as shown, and that the handle and/or plunger may comprise more, or fewer, components.

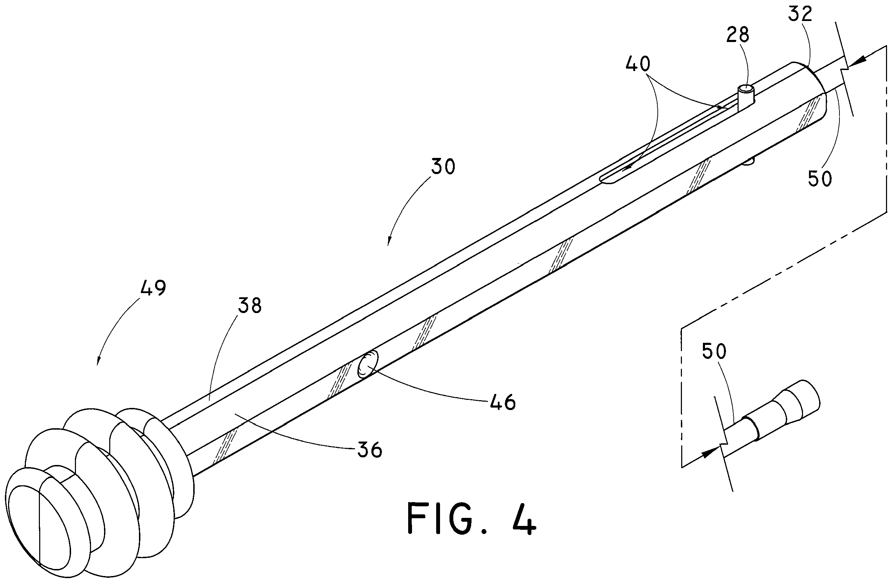

Plunger halves 36, 38 are shown in a disengaged condition in Fig. 3, and are shown in an engaged condition in Fig. 4. Handle 12 has been removed from Fig. 4, in order to better illustrate engagement of the plunger halves. Plunger halves 36, 38 may be engaged in any suitable fashion capable of forming a secure connection therebetween, such as via a screw connection or a snap connection. As shown in Fig. 3, plunger halves 36, 38 each have a cut-out portion 37, 39 along their respective lengths. When halves 36, 38 are engaged, cut-out portions 37, 39 define a channel 40 (Fig. 4) along the distal length of the plunger. The elongated interior of plunger 30 includes a first pathway 48 and a second pathway 44. First pathway 48 extends along the major length of the plunger. Second pathway 44 extends from the distal end of channel 40 to plunger distal end 32. The proximal end of outer sleeve 50 is fixedly received in pathway 44, e.g., by bonding with a compatible adhesive agent, such as an epoxy or glue. As a result, outer sleeve 50 is axially movable in correspondence with plunger 30.

Plunger 30 further includes an indent 46 proximal of channel 40. Indent 46 extends transversely through the proximal length of plunger 30, and is sized to receive ball 59 of ball plunger 58, as described herein. Preferably, plunger 30 also includes a knob 49 or like structure at its proximal end to facilitating grasping and control of the plunger during use of device 10. When present, knob 49 may be formed in any shape to facilitate grasping by the operator of the device.

Handle halves 18, 20, are shown in a disengaged condition in Fig. 3. Handle halves 18, 20 may be engaged in any suitable fashion capable of forming a secure connection between the respective halves, such as via a screw connection or a snap connection. As best shown in Fig. 3, handle halves 18, 20 preferably include respective large diameter proximal portions 24, 25, and smaller diameter distal portions 26, 27 extending in a distal direction therefrom. In the embodiment shown, small diameter distal portions 26, 27 are formed such that they have a grooved exterior. The grooved exterior is believed to facilitate handling of the re-positioning device, but is not otherwise essential to operation of the re-positioning device.

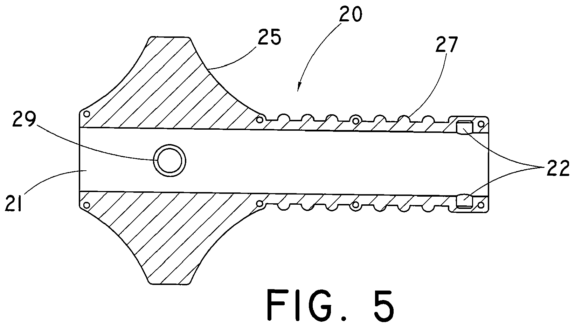

Handle halves 18, 20 also include respective longitudinal grooves 19, 21 along an internal surface thereof. Grooves 19, 21 are sized and arranged such that when handle halves 18, 20 are engaged to form handle 12, a longitudinal passageway is defined through the axial center of handle 12. As shown in Figs. 1-3, this longitudinal passageway is sized to receive plunger 30, and to permit relative movement between the plunger and handle along the passageway. Grooves 19, 21 are generally of constant diameter along the length of the groove, except for an extended diameter portion at the distal end of the respective handle half. Fig. 5 is a side view of the interior surface of handle half 20, illustrating extended diameter portion 22. Each handle half 18, 20 includes an extended diameter portion 22 as shown, such that when the handle halves are engaged as described, the respective extended diameter portions define an extended diameter segment along the passageway through handle 12. When handle halves 18, 20 are engaged, this extended diameter segment receives respective ends of capture pin 28, as described herein.

One of handle halves 18, 20 is fitted with a detent mechanism at the large diameter portion thereof. Detent mechanisms are known in the art as having the capacity to temporarily, or permanently, restrict, pause, or otherwise arrest movement of a first (movable) article with reference to a second (fixed) article. In the preferred embodiment shown, the detent mechanism comprises a ball plunger 58 (Figs. 2, 3) fitted at large diameter portion 25 of handle half 20. Ball plungers are well known in the art, and are available, for example, from J.W. Winco Inc. (e.g., Short Press Fit Ball Plunger, Stainless Steel, part number G09/N1). Ball plungers typically comprise an outer body portion (Fig. 3), encasing a spring (not shown) and a ball 59 therein. The ball 59 is biased by the spring through an opening at one end of the ball plunger.

Ball plunger 58 is sized and arranged such that the closed end of the ball plunger is received (e.g., by a press fit) into a corresponding hole 29 (Fig. 5) in the large diameter portion 25 of handle half 20. Ball 59 is biased transversely into the longitudinal passageway defined by handle grooves 19, 21. Ball plunger 58 is further sized and aligned such that ball 59 is received in plunger indent 46 during relative movement between plunger 30 and handle 12, as further described herein. Ball plungers are known in the art, and further description is not necessary for an understanding of the present invention. Those skilled in the art will appreciate that alternative devices capable of functioning in the manner of ball plunger 58 to selectively restrict, pause or otherwise arrest relative movement between the plunger and handle may be substituted for the detent device described herein with only minor modification of the overall structure.

As best shown in Figs. 2 and 3, a drive collar 41 is received interiorly of plunger 30. Drive collar 41 has a larger diameter proximal portion 42 received in first pathway 48, and a smaller diameter distal portion 43 extending distally from larger diameter portion 42 along second pathway 44. The proximal end of inner sleeve 60 is fixedly received, e.g., via crimping, over drive collar smaller diameter portion 43. The distal end of inner sleeve 60 extends in the distal direction interiorly of outer sleeve 50.

Drive collar larger diameter proximal portion 42 includes an aperture 45 extending transversely therethrough. A capture pin 28 is received in aperture 45. As best shown in Figs. 3 and 6-9, capture pin 28 is sized and arranged such that its respective axial ends are received in the extended diameter distal segment defined by extended diameter portions 22 of the handle halves 18, 20 as described above. The respective ends of capture pin 28 are thus positioned to ride along the length of plunger channel 40 during relative movement between handle 12 and plunger 30, as described herein. As best shown in Fig. 4, capture pin 28 acts as a stop mechanism for limiting relative axial movement between handle 12 and plunger 30 to a length defined by the length of channel 40.

Those skilled in the art will appreciate that the components described herein can be formed from conventional materials known to have the strength and stability for the respective purposes described. Thus, for example, the handle 12 and plunger 30 can be formed from a high strength polymeric material, such as the acetal resin DELRIN®. The drive collar, capture pin, outer sleeve, inner sleeve, and ball plunger can be formed from metal or metal alloys, such as stainless steel.

The manipulator mechanism cylinder, and manipulator arms, can also be formed from metal or metal alloys, such as stainless steel. The manipulator arms are structured such that they are capable of controllably encircling and manipulating the lead. The various bends and angles to which the manipulator arms may be adjusted to enable capture of a lead give the manipulator arms an ability to reach and maneuver the lead, as described below. When the manipulator arms include filled terminal loops 85, 87 as described above, the loops protect against entanglement of wires or lead coils within the loops, and also minimize the possibility of vessel wall damage. Preferably, the loops are filled in a manner that generally defines a dome. The bends, domed loops, and/or angles as specified enhance the ability of the device to safely manipulate and capture the lead, and to controllably maintain capture while maneuvering along the lead.

The inner and outer sleeves described herein may be formed from conventional biocompatible materials known for such purposes in the medical arts. As with the components described above, the inner and outer sleeves may also be formed of metals and metal alloys, such as stainless steel. Alternatively, the inner and outer sleeves may be formed from polymeric materials such polypropylene, polyurethane, polyethylene, nylon, PTFE, and the like. If desired, the sleeves can be reinforced along their length, or a segment of their length, with conventional reinforcing materials, such as a coil or a braid. Such reinforcements are well known in the medical arts, and are typically formed from a metal or metal alloy. If desired, selected portions of one or both of the sleeves, such as the distal tip portion, can be provided with means for x-ray or fluoroscopic vision. Such means are well known in the art, and may include, for example, the incorporation of a radiopaque band, or the inclusion of radiopaque particles in the selected portion. As still another alternative, a polymeric sleeve can be provided with a tip formed of a metal or metallic alloy to provide such visibility. Increased visibility of the tip may be beneficial because it allows the operator to determine the location of the tip at a particular point in time, and also provides the operator with the ability to track the position and orientation of the tip with reference to the lead body.

The respective dimensions of the components may be varied for a particular application of the positioning device. For example, in one non-limiting embodiment for use in positioning a cardiac lead as described, the handle may have dimensions of 3 in. [7.62 cm] Ig. x 0.75 in. [1.9 cm] OD (2 in. OD [5.1 cm] at proximal end). The plunger may have dimensions of 6.5 in. [16.5 cm] Ig. x 0.5 in. [1.27 cm] OD (1.5 in. [3.81 cm] OD at proximal end). The drive collar may have dimensions of 0.5 in. [1.3 cm] Ig. x 0.085 in. [0.22 cm] OD (smaller diameter portion of drive collar) and 0.25 in. [0.635 mm] OD (larger diameter portion of drive collar). The capture pin may have dimensions of 0.625 in. [1.59 cm] Ig. x. 0.125 in. [0.32 cm] OD. The outer sleeve may have dimensions of 12 in. [30.5 cm] Ig. x 0.134 in. [0.34 cm] OD x 0.114 in. [0.29 cm] ID. The inner sleeve may have dimensions of 12 in. [30.5 cm] Ig. x 0.109 in. [0.28 cm] OD x 0.085 in. [.22 cm] ID. The manipulator arms may have dimensions of 0.025 in. [0.064 cm] OD x 2 in. [5.1 cm] Ig. x 0.75 in. [1.9 cm] width.

Those skilled in the art will appreciate that these dimensions are only intended to represent the dimensions for one particular embodiment, and that the dimensions of any or all of the respective components may be varied as desired for a particular application. The components may be formed by well-known techniques, such as molding (e.g., handle and plunger), machining (e.g., drive collar and capture pin), welding and drawing (e.g., outer and inner sleeves), and wire forming (e.g., manipulator arms).

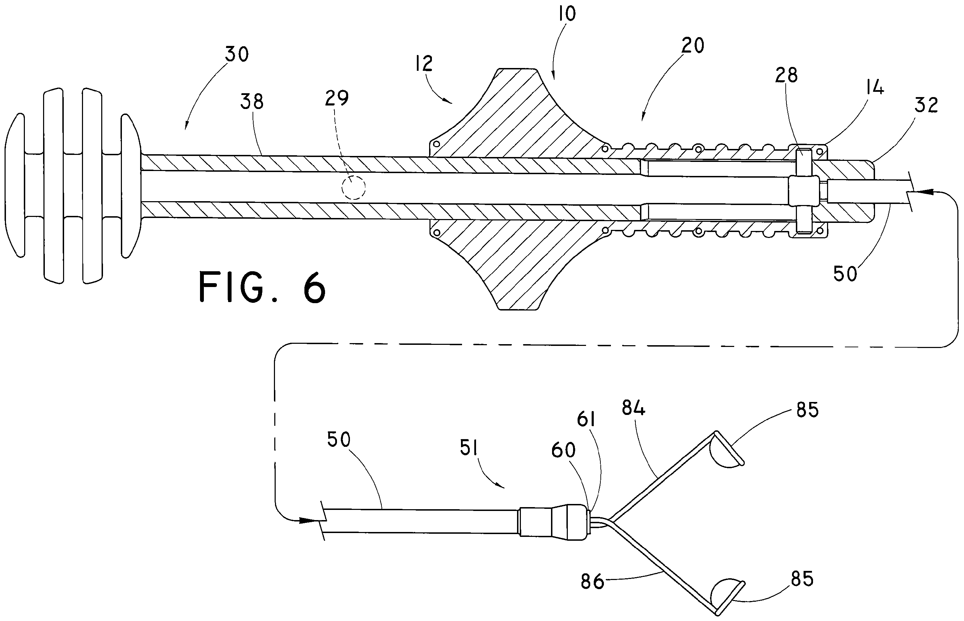

Operation of positioning device 10 will now be described, with primary reference being made to Figs. 6-9. Figs. 6-9 illustrate side views of positioning device 10, at various stages of relative movement between handle 12 and plunger 30. Handle half 18 and plunger half 36 have been removed from the views of Figs. 6-9 for ease of illustration of the internal elements.

Fig. 6 illustrates positioning device 10 in the initial, or "at rest", position. In this position, plunger 30 is fully retracted relative to handle 12. Capture pin 28 is positioned at the distal end of plunger channel 40. In this retracted position, distal end 51 of outer sleeve 50 is also retracted in a proximal direction relative to inner sleeve distal end 61, whereby manipulator arms 84, 86 are freely movable.

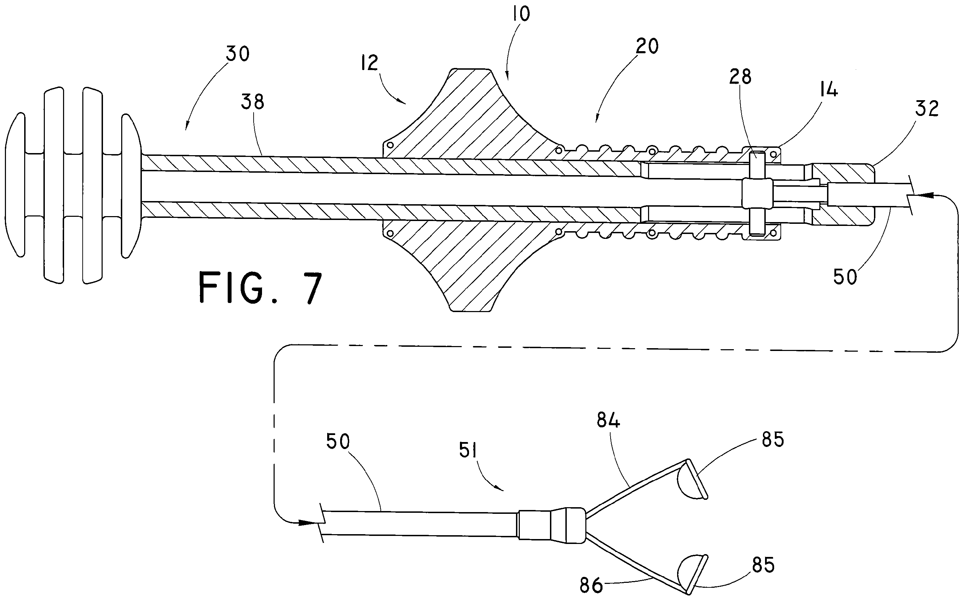

In order to manipulate arms 84, 86, in a manner such that a cardiac lead can be captured between the arms, plunger 30 is moved in the distal direction relative to handle 12, as shown in Fig. 7. This relative movement may be accomplished by urging plunger 30 in a distal direction relative to handle 12 in the nature of the plunger of a conventional syringe. Alternatively, the relative movement can be achieved by moving handle 12 in a proximal direction relative to plunger 30. For purposes of illustration only, this movement will be described herein as a distal movement of the plunger. Distal movement of plunger 30 causes corresponding distal movement of outer sleeve 50 relative to inner sleeve 60, as described. Movement of outer sleeve distal end 51 over a length of manipulator arms 84, 86 causes the manipulator arms to lose a portion of their free movement as shown. During this relative movement, ball plunger 58 (not shown) rides along the side of plunger half 38, in a manner such that ball 59 is biased inwardly, so that it remains substantially enclosed within the body of ball plunger 58.

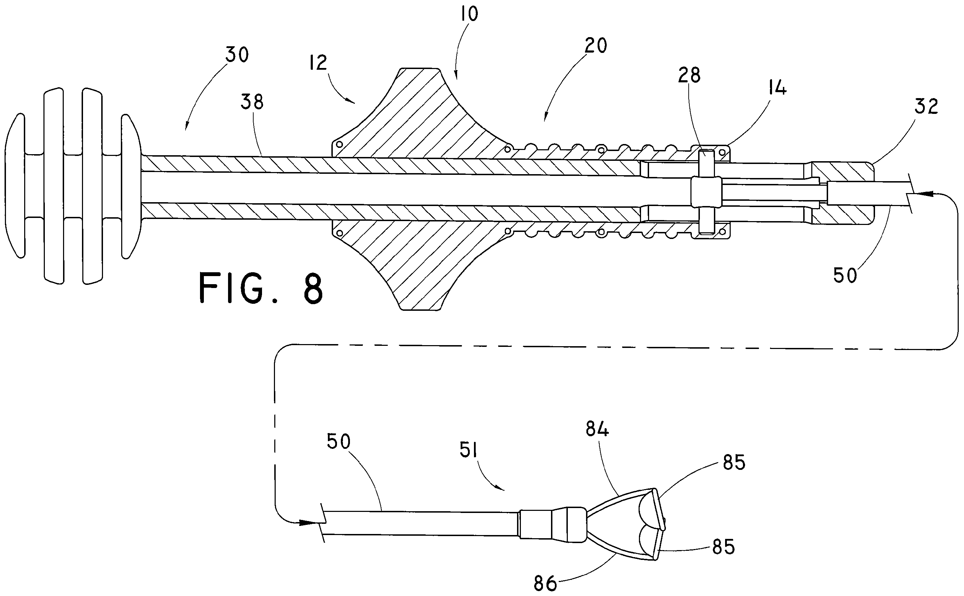

In the view of Fig. 8, plunger 30 has been further advanced in the distal direction relative to the view of Fig. 7. In this position, indent 46 (not shown) reaches ball plunger 58. This may perhaps be best understood by visualizing the relative position of ball plunger 58 and indent 46 as shown in Figs. 2 and 3, and envisioning further distal movement of plunger 30 until indent 46 reaches ball plunger 58. At this time, ball 59 springs outwardly and becomes seated in indent 46, such that further distal movement of plunger 30 is resisted.

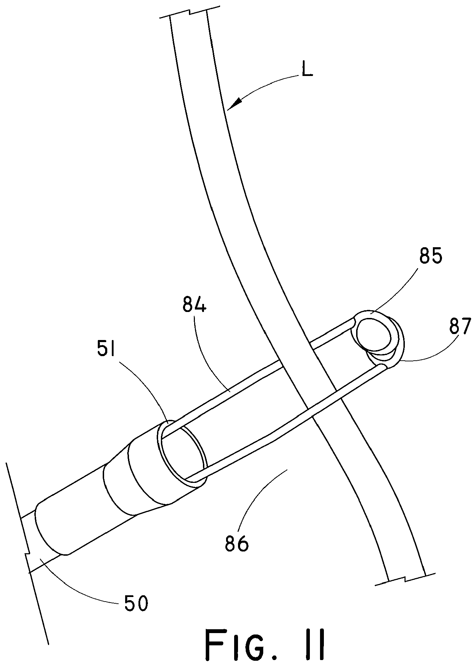

As a consequence of this distal movement of plunger 30, outer sleeve 50 has been further advanced distally over manipulator arms 84, 86, when compared to the position of Fig. 7, such that the manipulator arms meet in a semi-lock capture position as shown. In this position, the manipulator arms 84, 86, and more particularly, the terminal loops 85, 87 of the manipulator arms, are substantially engaged such that the cardiac lead may be captured between the arms (Fig. 11). At this time, the relative position of the manipulator arms with reference to the sleeves is sufficient to retain, or capture, the lead as described, but does not lock or otherwise prevent at least some sliding or other movement of the captured lead relative to the manipulator arms. As a result, the distal end of re-positioning device 10 remains movable along the lead, without losing capture of the lead, as described herein.

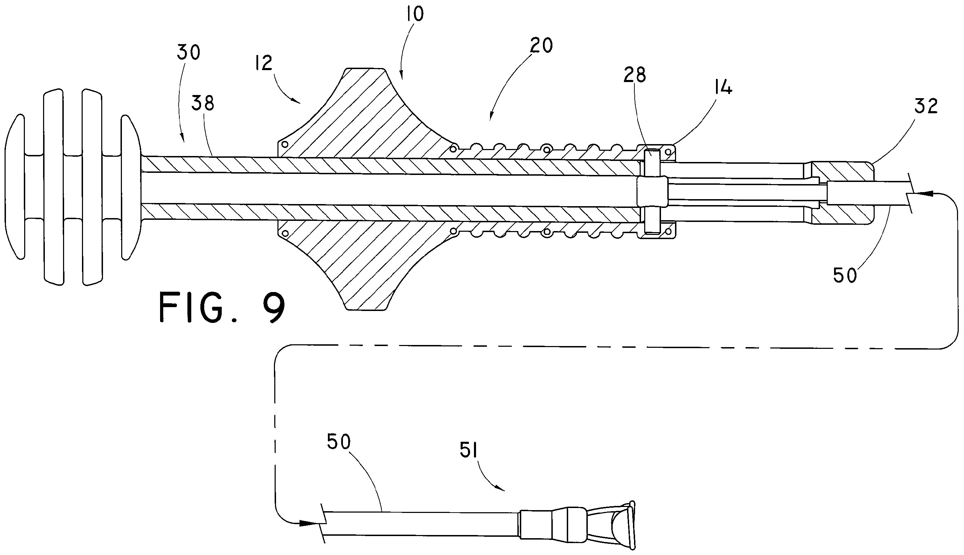

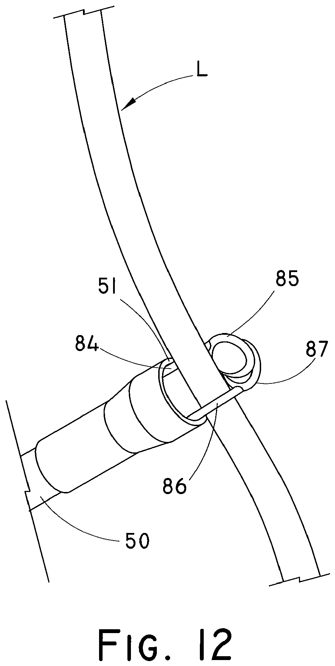

Plunger 30 is then further urged in the distal direction to overcome the resistance resulting from the capture of ball 59 in indent 46. Once this resistance is overcome, plunger 30 is further advanced until capture pin 28 abuts the proximal end of channel 40. This fully engaged capture position is shown in Fig. 9. At this point, the plunger cannot be further advanced in the distal direction. Outer sleeve 50 has been fully advanced over inner sleeve 60, and the lead is captured and locked (Fig. 12).

Those skilled in the art will appreciate that activation of the manipulator arms need not necessarily result from relative movement of outer and inner sleeves utilizing a plunger and handle as described. Other mechanisms for activating the manipulator arms may be substituted. For example, instead of utilizing an indent as a means of gauging position, a slotted rod mechanism with, e.g., a spring loaded button actuator may be substituted. This approach allows for automatic locking and manual unlocking from one position to another, to ensure that the device remained in the desired position until purposely unlocked by the user. A screw type mechanism could also be used to gauge and control position.

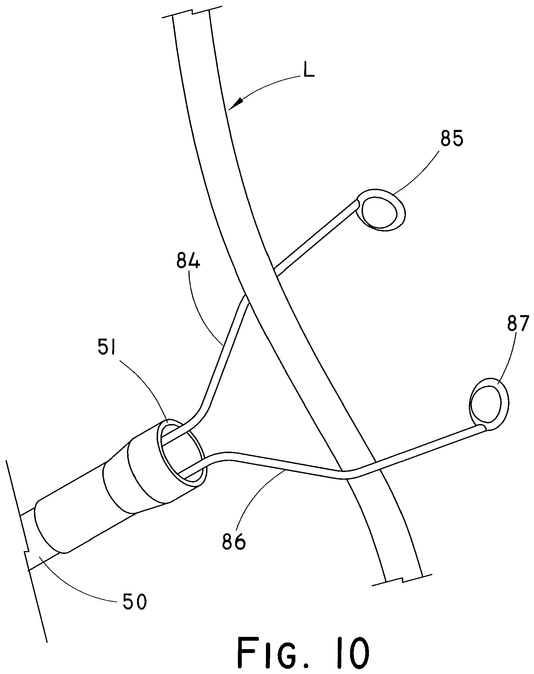

Figs. 10-12 are enlarged views of the positions of the manipulator arms 84, 86 in the positions depicted in Figs. 7-9, respectively, and showing a cardiac lead being captured and locked between the manipulator arms. Fig. 10 illustrates the arms positioned in a manner in which they retain a certain element of freedom of maneuverability as in Fig. 7, and illustrate a lead L that has been partially captured therebetween.

Fig. 11 illustrates the arms positioned in a manner in which they envelope the lead L in the semi-lock capture position as shown in Fig. 8. In this position, the manipulator arms 84, 86 are arranged in a suitable manner such that the cardiac lead may be captured between the arms, but the lead is not locked in a manner such that relative movement between the re-positioning device 10 and lead L would be prohibited. Thus, as stated above, when arranged as shown in Fig. 11, re-positioning device 10 remains movable along the lead, without losing capture of the lead. Fig. 12 illustrates the arms positioned in a manner such that the lead L is captured and locked, in the position shown in Fig. 9.

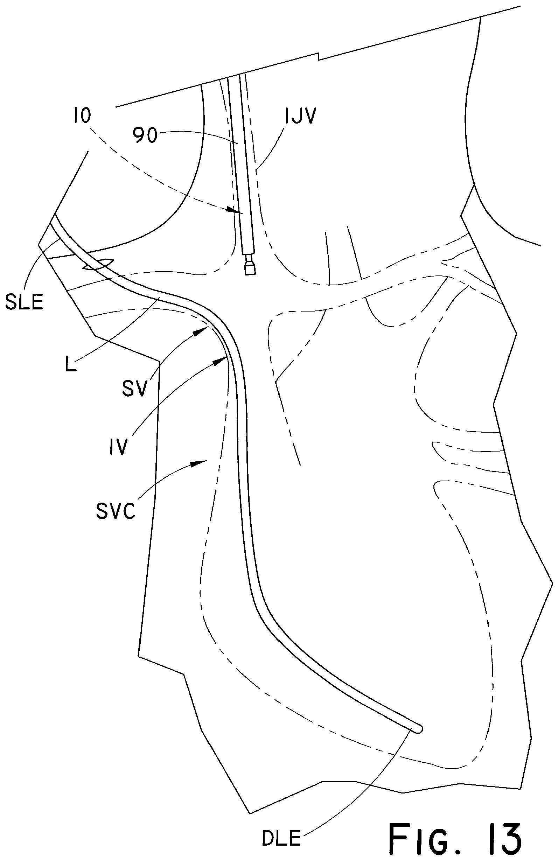

One example describing the use of positioning device 10 to reposition an implanted structure, such as cardiac lead L, to facilitate removal of the lead from the body of a patient will now be provided. This example is illustrated in Figs. 13-17.

In this example, a cardiac lead L extends from the implanted pacemaker or defibrillator along a curved pathway that extends through the subclavian vein (SV), the innominate (brachiocephalic) vein (IV), and into the superior vena cava (SVC). The distal lead end (DLE) is affixed in the heart. This arrangement is shown in Fig. 13. When the proximal end of the lead is severed from the pacemaker or defibrillator at SLE (severed lead end), it is common to track a conventional lead removal device over the severed end along this curved pathway, including the relatively extreme curve where the innominate vein joins the superior vena cava.

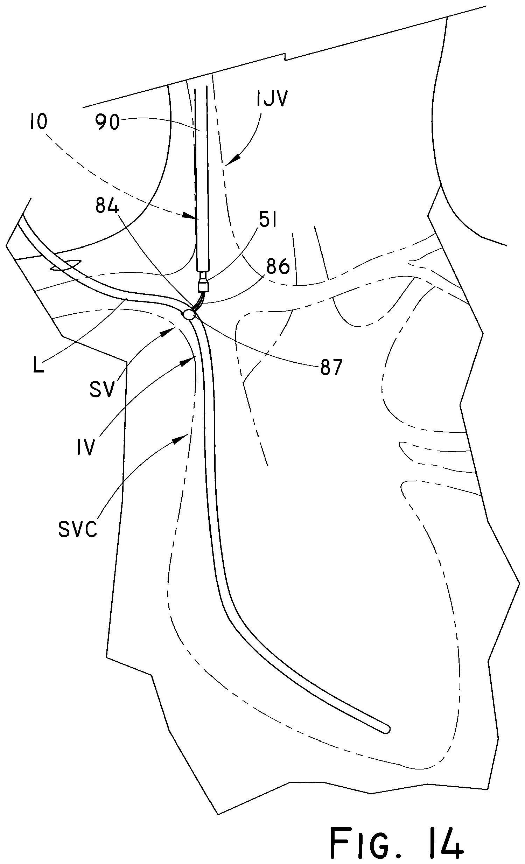

At times, however, it can be difficult to track over the lead along curves such as this with a conventional lead removal tool, if possible at all. Therefore, positioning device 10 may be utilized to adjust the position of lead L, in a manner that the lead is repositioned to a less curved pathway to facilitate removal of the lead. As shown in Fig. 14, positioning device 10 is inserted through an opening formed along a less curved vein, in this case, the internal jugular vein (IJV) of the patient. In the embodiment shown, lead 10 is inserted through an outer "work station" sheath 90 that has previously been inserted through the opening. Work station sheath 90 is preferably provided to control blood loss during insertion and manipulation of device 10. Work station sheath 90 may be a conventional introducer conduit having an inner diameter sufficient to receive device 10, and permit free movement therethrough of device 10. The work station sheath may be formed, e.g., of PTFE, and have a hemostasis valve at its proximal end. Introducer conduits such as sheath 90 are well known in the art for such purposes, and those skilled in the art can readily select an appropriate sheath for use with device 10.

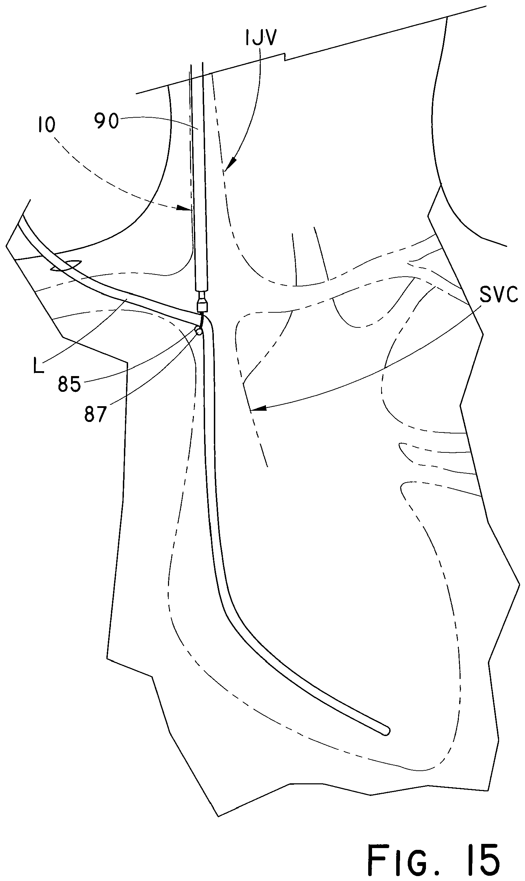

Device 10 is advanced through the work station sheath 90 along the internal jugular vein until the distal end of device 10 reaches a length of lead L. Device 10 is then maneuvered into position to capture the severed lead, as shown, e.g., in Fig. 14. Device 10 is then locked onto lead L, as shown in Fig. 15, according to the sequence discussed above.

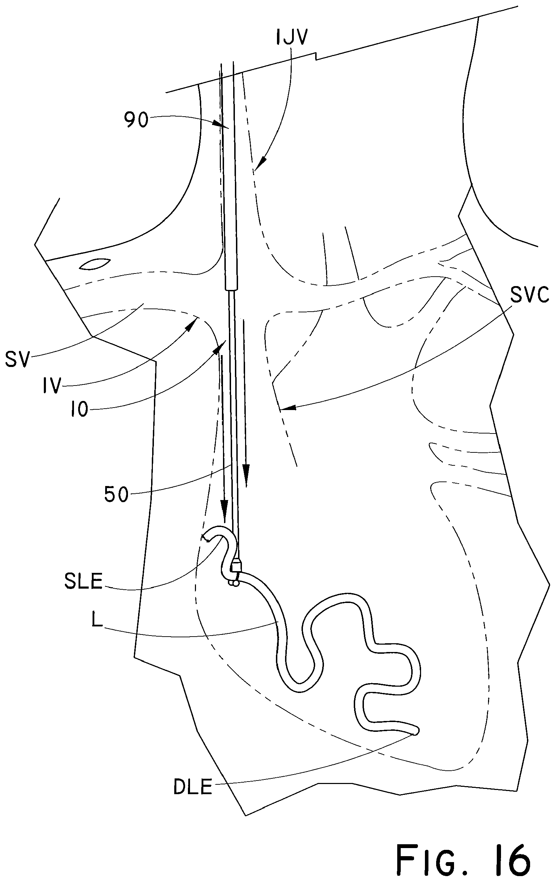

Device 10, and lead L locked onto device 10, are further advanced downwardly into the superior vena cava (SVC), preferably until severed lead end SLE has completely passed, or substantially completely passed, through the subclavian vein and the curve of the innominate vein (IV), and into the superior vena cava. This is indicated by the direction of the arrows in Fig. 16.

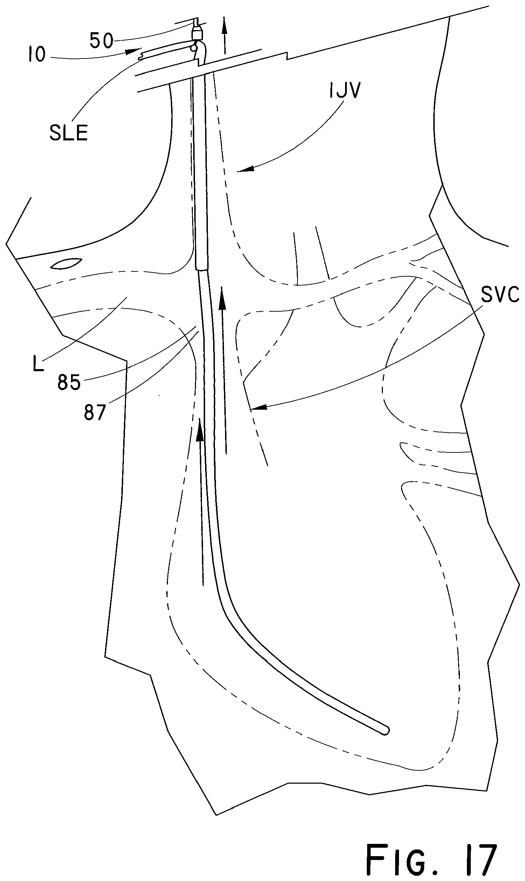

Device 10 and lead L are withdrawn to the extent possible through the relatively straight pathway of the internal jugular vein (IJV), as shown by the arrows in Fig. 17. At least the severed lead end SLE of lead L extends through the opening in the IJV. Depending upon the amount of encapsulating growth around lead L in the superior vena cava, only minimal withdrawal may be possible.

Following re-positioning, and any possible withdrawal, of the severed lead end SLE by device 10 as described, device 10 may be disengaged from lead L by reversing the steps described above with reference to Figs. 6-9. In this case, plunger 30 is withdrawn in the proximal direction relative to the handle, and the manipulating arms are released to the position shown in Figs. 7 and 10. The positioning device 10 may then be removed. A conventional lead removal tool (not shown) may then be positioned over the severed lead end SLE, and tracked in substantially linear fashion along the length of the lead in this pathway to free the lead from obstructions in well-known manner.

By utilizing device 10, the position of lead L has been adjusted in a manner such that the lead removal tool need not track the lead along a curved pathway, such as the curved pathway defined by the innominate vein and the superior vena cava. Rather, the lead removal tool tracks the lead over a fairly straight pathway defined by the internal jugular vein, as described and shown, e.g., in Figs. 16-17. This contrasts with the curved pathways that must be traversed in the techniques practiced in the art. Since the lead removal tool need not be capable of traversing the curved passageway, a more robust lead removal tool may be utilized if desired. Examples of robust lead removal tools include tools having a greater stiffness than tools that must traverse more severe bends in the vasculature, as well as tools capable of utilizing non-mechanical removal techniques (e.g., laser extraction and radio frequency extraction) to free the lead from encapsulating tissue.

Although positioning device 10 has been primarily described herein with regard to one possible use, i.e., for adjusting a position of a cardiac lead L implanted in a particular curved pathway to facilitate removal of the lead, those skilled in the art will appreciate that use of device 10 is not restricted to the specific use described in the examples. Rather, the device may be utilized in other instances in which it is desired to remove a structure implanted in biological tissue, and in which it would otherwise be necessary to track a removal tool along a severely curved, somewhat curved, or even a noncurved, pathway. The nature of the device with regard to its ability to capture, hold, push or pull lends itself to any application where these characteristics would be beneficial. The fact that the manipulating arms can encircle a structure even though that structure may be fixed at both ends also lends itself to other applications, e.g., as a tool for use in laparoscopic applications.

Those skilled in the art will appreciate that the manipulator mechanism need not necessarily be sized for intra-jugular access, and that a longer, flexible shaft (that can be used in either femoral access applications or in IJ access applications in which non-longitudinal (non-straight) distal tip positioning is required) can be substituted.

The foregoing detailed description should be regarded as illustrative rather than limiting, and it should be understood that the following claims, define the scope of this invention.

## Claims

1. A device (10) for adjusting a position of an elongated structure (L) implanted in biological tissue to facilitate removal thereof, comprising:
an outer sleeve (50) having a proximal end and a distal end (51);
an inner sleeve (60) received in the outer sleeve and movable relative thereto, the inner sleeve having a proximal end and a distal end (61);
a manipulator mechanism (80) engaged with the inner sleeve distal end and extending in a distal direction from said inner sleeve distal end, the manipulator mechanism structured and arranged for capture of the elongated structure, and for locking the elongated structure to the device upon relative movement of the inner and outer sleeves;
a handle (12) engaged with the inner sleeve proximal end; and
a plunger (30) engaged with the outer sleeve proximal end, the plunger engaged with the handle and movable relative thereto, the plunger and handle aligned such that upon said relative movement therebetween the outer sleeve advances distally relative to the inner sleeve in a manner such that the manipulator mechanism is maneuverable to capture the implanted elongated structure, whereby the position of the elongated structure may be controllably adjusted to facilitate removal thereof from the biological tissue;
wherein the manipulator mechanism comprises a pair of manipulator arms (84, 86), said manipulator arms movable upon said relative movement between said plunger and handle between a substantially open position when said plunger is at a maximum proximal extension relative to said handle and said elongated structure is freely movable relative to said manipulator arms, and a substantially locked position when said plunger is at a maximum distal extension relative to said handle such that movement of said elongated structure relative to said manipulator arms is substantially prevented; and
wherein said handle includes a longitudinal passageway extending therethrough, and a length of said plunger is receivable in said passageway and aligned therein for said relative movement therebetween, said plunger including an indent (46) along said plunger length receivable in said passageway, and said handle including a detent mechanism (58), said detent mechanism having a portion (59) thereof sized and aligned along said passageway such that said detent portion is receivable in said indent for resisting further relative movement between said plunger and said handle, wherein said manipulator arms are arranged for capture of said elongated structure in a manner that permits controlled movement of said elongated structure between said manipulator arms when said detent portion is received in said indent.

2. A device as claimed in claim 1, wherein said detent mechanism comprises a ball plunger (58), wherein said receivable portion comprises a ball (59) receivable in said indent, said detent mechanism further comprising a bias member for urging said ball into said indent.

3. A device as claimed in claim 1, wherein said plunger includes a channel (40) extending axially therealong, said channel having a length, said device further including a stop mechanism (28) movable relative to said channel for restricting said relative movement between said plunger and said handle to said channel length.

4. A device as claimed in claim 3, wherein said stop mechanism comprises a capture pin (28), said capture pin having first and second axial ends engaged with said longitudinal passageway of said handle, said capture pin positioned such that said axial ends extend through said channel for said restriction of relative movement between said plunger and said handle.

5. A device as claimed in claim 4, further comprising a drive collar (41) received in an interior pathway of said plunger, said drive collar having an aperture (45) extending therethrough and aligned such that said capture pin extends through said drive collar aperture.

6. A device as claimed in claim 5, wherein said drive collar has a large diameter proximal portion (42) and a small diameter distal portion (43), said large diameter proximal portion received in said interior pathway and having said aperture therethrough, said small diameter distal portion engaged with said inner sleeve proximal end.

7. A device as claimed in claim 1, wherein said manipulator arms comprise respective elongated wire members (84, 86) extending distally from said inner sleeve.

8. A device as claimed in claim 7, wherein said wire members terminate in respective loops (85, 87), said loop of one manipulator arm substantially engageable with the loop of the other manipulator arm when said detent is received in said indent for capturing said elongated structure therebetween.

9. A device as claimed in claim 8, wherein said loops include a filler material therein, which increases visibility and tracking of the manipulator arms or ensures that the elongated structure does not inadvertently become trapped within said loops.

10. A device as claimed in claim 4, wherein said plunger comprises first and second plunger halves (36, 38), each of said plunger halves comprising a cut-out portion (37, 39) along a length thereof, said cut-out portions defining said channel (40) upon joinder of said first and second plunger halves.

11. A device as claimed in claim 10, wherein said handle comprises first and second handle halves (18, 20), each of said handle halves including an extended diameter portion (26, 27), said extended diameter portions configured and arranged such that upon joinder of said first and second handle halves the extended diameter portions define an extended diameter segment along said handle passageway, said extended diameter segment dimensioned to receive respective axial ends of said capture pin (28).

## Patentansprüche

1. Vorrichtung (10) zur Anpassung einer Position einer in biologischem Gewebe implantierten länglichen Struktur (L) zur leichteren Entfernung, umfassend:
eine Außenhülse (50) mit einem proximalen Ende und einem distalen Ende (51);
eine Innenhülse (60), die in der Außenhülse aufgenommen ist und bezüglich dieser beweglich ist, wobei die Innenhülse ein proximales Ende und ein distales Ende (61) aufweist;
einen Manipulatormechanismus (80), der mit dem distalen Ende der Innenhülse in Eingriff ist und sich in eine distale Richtung vom distalen Ende der Innenhülse erstreckt, wobei der Manipulatormechanismus eine Struktur und Anordnung zum Ergreifen der länglichen Struktur und zum Arretieren der länglichen Struktur auf der Vorrichtung bei einer relativen Bewegung der Innen- und Außenhülse aufweist;
einen Griff (12), der mit dem proximalen Ende der Innenhülse in Eingriff ist; und
einen Kolben (30), der mit dem proximalen Ende der Außenhülse in Eingriff ist, wobei der Kolben mit dem Griff in Eingriff ist und im Verhältnis dazu beweglich ist, wobei der Kolben und der Griff derart ausgerichtet sind, dass bei der besagten relativen Bewegung zwischen ihnen die Außenhülse distal bezüglich der Innenhülse auf eine solche Weise vorgeschoben wird, dass der Manipulatormechanismus zum Ergreifen der implantierten länglichen Struktur manövrierbar ist, wobei die Position der länglichen Struktur kontrollierbar eingestellt werden kann, damit sie leichter aus dem biologischen Gewebe entfernt werden kann;
wobei der Manipulatormechanismus ein Paar Manipulatorarme (84, 86) umfasst, wobei die Manipulatorarme bei der besagten relativen Bewegung zwischen dem Kolben und dem Griff zwischen einer im Wesentlichen geöffneten Stellung, wenn der Kolben bezüglich des Griffs maximal proximal ausgestreckt ist und die längliche Struktur bezüglich der Manipulatorarme frei beweglich ist, und einer im Wesentlichen verriegelten Stellung, wenn der Kolben bezüglich des Griffs maximal distal ausgestreckt ist, bewegbar sind, derart, dass eine Bewegung der länglichen Struktur bezüglich der Manipulatorarme im Wesentlichen verhindert wird; und
wobei der Griff einen sich durch ihn hindurch erstreckenden langgestreckten Durchgang aufweist und eine Länge des Kolbens in dem Durchgang aufnehmbar und darin für die besagte relative Bewegung dazwischen ausrichtbar ist, wobei der Kolben eine Vertiefung (46) entlang der im Durchgang aufnehmbaren Kolbenlänge aufweist, und der Griff einen Sperrmechanismus (58) aufweist, wobei der Sperrmechanismus einen Abschnitt (59) aufweist, der eine solche Größe hat und entlang des Durchgangs so ausgerichtet ist, dass der Sperrabschnitt in der Vertiefung aufnehmbar ist, um einer weiteren relativen Bewegung zwischen dem Kolben und dem Griff zu widerstehen, wobei die Manipulatorarme zum Ergreifen der länglichen Struktur ausgelegt sind, auf eine Weise, die eine kontrollierte Bewegung der länglichen Struktur zwischen den Manipulatorarmen gestattet, wenn der Sperrabschnitt in der Vertiefung aufgenommen ist.

2. Vorrichtung nach Anspruch 1, wobei der Sperrmechanismus einen Kugelkolben (58) umfasst, wobei der aufnehmbare Abschnitt eine Kugel (59) umfasst, die in der Vertiefung aufgenommen werden kann, wobei der Sperrmechanismus ferner ein Vorspannelement umfasst, um die Kugel in die Vertiefung zu drängen.

3. Vorrichtung nach Anspruch 1, wobei der Kolben einen sich axial dort entlang erstreckenden Kanal (40) aufweist, wobei der Kanal eine Länge aufweist, die Vorrichtung ferner einen Anschlagmechanismus (28) aufweist, der bezüglich des Kanals beweglich ist, um die relative Bewegung zwischen dem Kolben und dem Griff auf die Kanallänge zu begrenzen.

4. Vorrichtung nach Anspruch 3, wobei der Anschlagmechanismus einen Fangstift (28) umfasst, wobei der Fangstift erste und zweite axiale Enden aufweist, die mit dem langgestreckten Durchgang des Griffs in Eingriff stehen, wobei der Fangstift derart platziert ist, dass sich die axialen Enden zur Begrenzung der relativen Bewegung zwischen dem Kolben und dem Griff durch den Kanal erstrecken.

5. Vorrichtung nach Anspruch 4, ferner umfassend einen Antriebskragen (41), der in einem inneren Durchgang des Kolbens aufgenommen ist, wobei der Antriebskragen eine sich durch ihn hindurch erstreckende Öffnung (45) aufweist, die so ausgerichtet ist, dass sich der Fangstift durch die Öffnung des Antriebskragens erstreckt.

6. Vorrichtung nach Anspruch 5, wobei der Antriebskragen einen proximalen Abschnitt (42) mit einem großen Durchmesser und einen distalen Abschnitt (43) mit einem kleinen Durchmesser aufweist, wobei der proximale Abschnitt mit einem großen Durchmesser in dem inneren Durchgang aufgenommen ist und die durch ihn hindurch gehende Öffnung aufweist, wobei der distale Abschnitt mit einem kleinen Durchmesser mit dem proximalen Ende der Innenhülse in Eingriff ist.

7. Vorrichtung nach Anspruch 1, wobei die Manipulatorarme jeweilige langgestreckte Drahtelemente (84, 86) umfassen, die sich distal von der Innenhülse erstrecken.

8. Vorrichtung nach Anspruch 7, wobei die Drahtelemente in jeweiligen Schleifen (85, 87) enden, wobei die Schleife eines Manipulatorarms im Wesentlichen mit der Schleife des anderen Manipulatorarms in Eingriff gebracht werden kann, wenn die Sperre in der Vertiefung aufgenommen wird, um die längliche Struktur dazwischen zu ergreifen.

9. Vorrichtung nach Anspruch 8, wobei die Schleifen ein Füllmaterial darin aufweisen, das die Sichtbarkeit und Verfolgung der Manipulatorarme erhöht oder sicherstellt, dass die längliche Struktur nicht versehentlich in den Schleifen eingeklemmt wird.

10. Vorrichtung nach Anspruch 4, wobei der Kolben erste und zweite Kolbenhälften (36, 38) umfasst, wobei jede der Kolbenhälften einen Ausschnittabschnitt (37, 39) entlang einer Länge davon umfasst, wobei die Ausschnittabschnitte nach der Verbindung der ersten und zweiten Kolbenhälften den Kanal (40) definieren.

11. Vorrichtung nach Anspruch 10, wobei der Griff erste und zweite Griffhälften (18, 20) umfasst, wobei jede der Griffhälften einen Abschnitt (26, 27) mit verlängertem Durchmesser aufweist, wobei die Abschnitte mit verlängertem Durchmesser derart ausgelegt und angeordnet sind, dass nach der Verbindung der ersten und zweiten Griffhälften die Abschnitte mit verlängertem Durchmesser ein Segment mit verlängertem Durchmesser entlang des Griffdurchgangs definieren, wobei das Segment mit verlängertem Durchmesser Abmessungen zur Aufnahme jeweiliger Enden des Fangstifts (28) aufweist.

## Revendications

1. Dispositif (10) pour régler la position d'une structure allongée (L) implantée dans un tissu biologique pour faciliter l'extraction de celle-ci, comprenant :
un manchon extérieur (50) comportant une extrémité proximale et une extrémité distale (51) ;
un manchon intérieur (60) accueilli dans le manchon extérieur et mobile par rapport à celui-ci, le manchon intérieur comportant une extrémité proximale et une extrémité distale (61) ;
un mécanisme manipulateur (80) en prise avec l'extrémité distale du manchon intérieur et s'étendant dans une direction distale depuis ladite extrémité distale du manchon intérieur, le mécanisme manipulateur étant structuré et agencé pour se saisir de la structure allongée et pour verrouiller la structure allongée au dispositif lors d'un mouvement relatif des manchons intérieur et extérieur ;
une poignée (12) en prise avec l'extrémité proximale du manchon intérieur ; et
un piston (30) en prise avec l'extrémité proximale du manchon extérieur, le piston étant en prise avec la poignée et mobile par rapport à celle-ci, le piston et la poignée étant alignés de telle sorte que lors dudit mouvement relatif entre eux le manchon extérieur avance distalement par rapport au manchon intérieur de telle sorte qu'on peut manoeuvrer le mécanisme manipulateur pour se saisir de la structure allongée implantée, moyennant quoi on peut régler de façon contrôlable la position de la structure allongée pour faciliter l'extraction de celle-ci du tissu biologique,
dans lequel le mécanisme manipulateur comprend une paire de bras manipulateurs (84, 86), lesdits bras manipulateurs étant mobiles lors dudit mouvement relatif entre ledit piston et ladite poignée, entre une position pratiquement ouverte, quand ledit piston est au maximum d'extension proximale par rapport à la poignée et que ladite structure allongée est librement mobile par rapport auxdits bras manipulateurs, et une position pratiquement verrouillée, quand ledit piston est au maximum d'extension distale par rapport à la poignée de telle sorte qu'est pratiquement empêché tout mouvement de ladite structure allongée par rapport auxdits bras manipulateurs ; et
dans lequel ladite poignée comprend un passage longitudinal qui la traverse et une certaine longueur dudit piston peut être accueillie dans ledit passage et y être alignée pour ledit mouvement relatif entre eux, ledit piston comprenant une entaille (46) dans ladite longueur de piston qui peut être accueillie dans ledit passage et ladite poignée comprenant un mécanisme d'encliquetage (58), ledit mécanisme d'encliquetage comportant une partie (59) de celui-ci qui est dimensionnée et alignée suivant ledit passage de telle sorte que ladite partie d'encliquetage peut être accueillie dans ladite entaille pour s'opposer à la poursuite du mouvement relatif entre ledit piston et ladite poignée, dans lequel lesdits bras manipulateurs sont agencés pour se saisir de ladite structure allongée d'une manière qui permette un mouvement contrôlé de ladite structure allongée entre lesdits bras manipulateurs quand ladite partie d'encliquetage est accueillie dans ladite entaille.

2. Dispositif selon la revendication 1, dans lequel ledit mécanisme d'encliquetage comprend un plongeur à bille (58), dans lequel ladite partie pouvant être accueillie est une bille (59) pouvant être accueillie dans ladite entaille, ledit mécanisme d'encliquetage comprenant outre un élément de sollicitation pour pousser ladite bille dans ladite entaille.

3. Dispositif selon la revendication 1, dans lequel ledit piston comprend un canal (40) s'étendant suivant son axe, ledit canal ayant une certaine longueur, ledit dispositif comprenant en outre un mécanisme de butée (28) mobile par rapport audit canal pour limiter à ladite longueur du canal ledit mouvement relatif entre ledit piston et ladite poignée.

4. Dispositif selon la revendication 3, dans lequel ledit mécanisme de butée comprend une clavette (28) de capture, ladite clavette de capture comportant une première et une seconde extrémité axiale en prise avec ledit passage longitudinal de ladite poignée, ladite clavette de capture étant placée de telle sorte que lesdites extrémités axiales traversent ledit canal pour ladite restriction du mouvement relatif entre ledit piston et ladite poignée.

5. Dispositif selon la revendication 4, comprenant en outre un collier d'entraînement (41) accueilli dans un passage intérieur dudit piston, ledit collier d'entraînement comportant une ouverture (45) le traversant et alignée de telle sorte que ladite clavette de capture traverse ladite ouverture de collier d'entraînement.

6. Dispositif selon la revendication 5, dans lequel ledit collier d'entraînement comporte une partie proximale (42) de grand diamètre et une partie distale (43) de petit diamètre, ladite partie proximale de grand diamètre étant accueillie dans ledit passage intérieur et comportant ladite ouverture le traversant, ladite partie distale de petit diamètre étant en prise avec ladite extrémité proximale du manchon intérieur.

7. Dispositif selon la revendication 1, dans lequel lesdits bras manipulateurs comprennent chacun un élément allongé (84, 86) en fil métallique s'étendant distalement depuis ledit manchon intérieur.

8. Dispositif selon la revendication 7, dans lequel lesdits éléments en fil métallique se terminent chacun par une boucle (85, 87), ladite boucle d'un bras manipulateur peut pratiquement entrer en prise avec la boucle de l'autre bras manipulateur pour saisir entre elles ladite structure allongée quand ladite partie d'encliquetage est accueillie dans ladite entaille.

9. Dispositif selon la revendication 8, dans lequel lesdites boucles contiennent un matériau de remplissage qui augmente la visibilité et améliore le suivi des bras manipulateurs ou garantit que la structure allongée ne se retrouve pas piégée par mégarde à l'intérieur desdites boucles.

10. Dispositif selon la revendication 4, dans lequel ledit piston comprend une première et une seconde moitié (36, 38) de piston, chacune desdites moitiés de piston comprenant une partie de découpe (37, 39) sur une certaine longueur de celles-ci, lesdites parties de découpe délimitant ledit canal (40) lorsqu'on assemble lesdites première et seconde moitiés de piston.

11. Dispositif selon la revendication 10, dans lequel ladite poignée comprend une première et une seconde moitié (18, 20) de poignée, chacune desdites moitiés de poignée comprenant une partie (26, 27) de diamètre étendu, lesdites parties de diamètre étendu étant configurées et agencées de telle sorte que, lorsqu'on assemble lesdites première et seconde moitiés de poignée, les parties de diamètre étendu définissent un segment de diamètre étendu le long dudit passage de poignée, ledit segment de diamètre étendu étant dimensionné pour accueillir les extrémités axiales respectives de ladite clavette (28) de capture.
